# EUROPÄISCHE PATENTANMELDUNG

(11) **EP 0 966 892 A2**
(43) Veröffentlichungstag der Anmeldung: **29.12.1999**
(21) Anmeldenummer: 99112197.1
(22) Anmeldetag: 24.06.1999
(51) Int. Cl.: A41D 13/12

(54) **Schutzbekleidungsartikel**

(30) Priorität: 24.06.1998 DE 19828172; 07.05.1999 DE 19921203
(71) Anmelder: Siewert, Ronald R., 40885 Ratingen (DE)
(72) Erfinder: Siewert, Ronald R., 40885 Ratingen (DE)
(74) Vertreter: Manitz, Finsterwald & Partner

(57) **Zusammenfassung**

Die Erfindung betrifft einen Schutzbekleidungsartikel zur zumindest teilweisen Verhinderung von Kratzverletzungen, insbesondere an gefährdeten Körperstellen bei Patienten, vornehmlich bei Kleinkindern und Säuglingen mit Neurodermitis. Der Schutzbekleidungsartikel zeichnet sich dadurch aus, daß der Artikel aus einer inneren, der gefährdeten Körperstelle zugewandten Lage und einer äußeren Lage besteht und daß die äußere Lage an der inneren Lage nur an Stellen im Randbereich der inneren Lage fixiert bzw. fixierbar und gegenüber der inneren Lage bei Kratzbewegungen des Patienten verschiebbar ist.

## Beschreibung

Die vorliegende Erfindung betrifft einen Schutzbekleidungsartikel zur zumindest teilweisen Verhinderung von Kratzverletzungen, insbesondere an gefährdeten Körperstellen bei Patienten, vornehmlich, jedoch nicht ausschließlich bei Kleinkindern und Säuglingen mit Neurodermitis.

Die Neurodermitis (Synonyme: endogenes Ekzem, atopisches Ekzem, atopische Dermatitis) ist ein Krankheitsbild, das zu den allergischen Erkrankungen gehört. Die Neurodermitis ist erblich und kann durch seelische Trauma verschlimmert oder verbessert werden. Im Winter oder Frühjahr wird häufig eine Verschlechterung des Krankheitsbildes beobachtet.

Die Erkrankung beginnt häufig im Säuglings- oder Kleinkindalter und zeigt sich mit Rötungen und Bläschen im Gesicht und auf dem behaarten Kopf (Milchschorf). Nach dem zweiten Lebensjahr entspricht das Bild im wesentlichen der Neurodermitis des Erwachsenen. Während im Kindesalter meist die Ellen- und Kniebeugen und nicht selten auch das Gesäß befallen sind, sind beim Erwachsenen vorwiegend das Gesicht, Hals, Nacken, Brust, Schulter und Gelenkbeugen betroffen. Die Haut ist an diesen Stellen chronisch entzündet. Hauptsymptom der Neurodermitis ist der quälende Juckreiz, häufig in Verbindung mit nächtlichen Juckkrisen. Auf den starken Juckreiz reagieren die Patienten mit intensivem Kratzen, da Schmerz häufig besser zu ertragen ist als Juckreiz. Kratzen bringt jedoch nur vorübergehende Linderung. Danach juckt es stärker als zuvor.

Ständiges Kratzen wiederum kann zu bakteriellen Infektionen der einzelnen Hautareale führen. Neurodermitis-Patienten zeigen Unverträglichkeiten gegenüber eine Reihe von Textilmaterialien, z.B. Wolle, Mohair und Angora, da diese Materialien aufgrund ihrer Faserstruktur entweder die Haut zusätzlich reizen oder zu wenig kühlende Luft an die entzündeten Hautstellen heranlassen und/oder durch unzureichende Abdünstung von Schweiß zu Wärmestau führen. Derzeit wird therapeutisch empfohlen, Kindern die Fingernägel kurz zu schneiden und nachts spezielle Overalls oder Baumwollhandschuhe anzuziehen, um schlimme Kratzspuren zu verhindern.

Aufgabe der vorliegenden Erfindung ist, Schutzbekleidungsartikel vorzusehen, die die Gefahr von Kratzverletzungen vorbeugen und im übrigen als hautsympathisch gelten. Weiterhin sollten solche Schutzbekleidungsartikel waschbar und wiederverwendbar sein.

Zur Lösung dieser Aufgabe wird erfindungsgemäß vorgesehen, daß der Artikel aus einer inneren, der gefährdeten Körperstelle zugewandten Lage und einer äußeren Lage besteht und daß die äußere Lage an der inneren Lage nur an Stellen im Randbereich der inneren Lage fixiert bzw. fixierbar und gegenüber der inneren Lage bei Kratzbewegungen des Patienten verschiebbar ist.

Wenn sich nun der Patient kratzt, werden lediglich im Prinzip die inneren und äußeren Lagen gegeneinander verschoben. Das Gehirn registriert den Kratzeffekt, der Linderung schafft, ohne daß es zu Verletzungen der Haut kommt, die üblicherweise durch das Kratzen mit den Fingernägeln verursacht werden.

Die erfindungsgemäßen Schutzbekleidungsartikel können sowohl als Schutzlagen, die an zu schützende Stellen angebracht werden können, ausgebildet sein, als auch in der Form von Bekleidungsartikeln, beispielsweise als Bekleidungsoberteile, Hosen, Overalls, Handschuhe etc., vorliegen, in welche an betreffende Stellen derartige Schutzlagen eingearbeitet sind.

Es kann sich bei diesem Schutzbekleidungsartikel um einen Handschuh handeln, und zwar dann, wenn die gefährdete Körperstelle die Hände selbst sind, die bei manchen Patienten zu den von starkem Juckreiz geplagten Bereichen gehören. Aber selbst bei Patienten, die an den Händen keinen Juckreiz spüren, ist es äußerst nützlich, wenn es sich bei dem Schutzbekleidungsartikel um einen Handschuh handelt mit einer zumindest im Bereich der Finger vorgesehenen inneren Lage und einer zumindest die Finger, ggf. die Hand, umfassenden äußere Lage und wenn die beiden Lagen zumindest im Fingerbereich bei Kratzbewegungen gegeneinander verschiebbar sind. In diesem Fall ist auch der Bereich der Fingerspitzen, von denen die Kratzbewegungen ausgehen, durch die zwei gegeneinander verschiebbaren Lagen sozusagen entschärft, da durch das gegenseitige Verschieben der zwei Lagen in diesem Bereich die aufkratzende Wirkung der Fingerspitzen wesentlich herabgesetzt wird. Es ist durch das Tragen von solchen Handschuhen ein doppelter Schutz möglich. Wenn man nämlich mit so geschützten Händen versucht, sich an einer anderen, gefährdeten, ebenfalls mit einem erfindungsgemäßen Schutzbekleidungsartikel geschützten Körperstelle zu kratzen, beispielsweise im Bereich der Knie- oder Ellenbogenbeuge, sind zwei bis vier gegeneinander verschiebbare Lagen im Spiel und zwar die innere und äußere Lage des Handschuhs und die innere und äußere Lage des Schutzbekleidungsartikels, der für die Kniebeuge bzw. für die Ellbogenbeuge benutzt wird.

Es wird bzw. werden üblicherweise eine Befestigungseinrichtung bzw. mehrere Befestigungseinrichtungen vorgesehen, um den Schutzbekleidungsartikel an der vorgesehenen Körperstelle zu halten. Gerade bei Kleinkindern verhindert dies, daß der Schutzbekleidungsartikel verrutscht und so die gefährdete Körperstelle freigibt. Es wird weiterhin verhindert, daß der Schutzbekleidungsartikel in Form eines Handschuhs vom Kleinkind leicht entfernt werden kann. Auch bei Erwachsenen sind solche Befestigungseinrichtungen nützlich, da das Kratzen in der Nacht teilweise unbewußt geschieht und die entsprechenden Befestigungseinrichtungen verhindern, daß der Patient die entsprechenden Schutzbekleidungsartikel ohne weiteres im Halbschlaf entfernt.

Die verwendeten Materialien, die vorzugsweise aus Polyester bestehen und später näher beschrieben werden, sind in hohem Maße luft- und wasserdampfdurchlässig. Sie bestehen weiterhin aus Fasern, deren Oberfläche so glatt ist, daß zusätzliche Reizungen der entzündeten Hautbereiche ausgeschlossen sind. Um die mit dem Schutzbekleidungsartikel geschützten Stellen nicht unnötig warm werden zu lassen, was auch zur Intensivierung des Juckreizes führen kann, wird die innere Lage vorzugsweise lediglich der entsprechenden gefährdeten Körperstelle gegenüber angeordnet, jedoch nicht so ausgebildet, daß sie das entsprechende Körperteil umfaßt. Beispielsweise soll bei einem Schutzbekleidungsartikel, der zum Schutz der Ellbogenbeuge verwendet wird, die innere Lage zwar der Ellbogenbeuge gegenüberliegen, das entsprechende Körperteil, d.h. das Ellbogengelenk, jedoch nicht umfassen.

Allerdings gibt es auch Stellen, wo es sinnvoll ist, daß die innere Lage der entsprechenden Körperstelle gegenüberliegt und das entsprechende Körperteil umfaßt. Ein Beispiel hierfür ist der Handschuh, und zwar gerade dann, wenn der Patient mit Juckreiz im Bereich des Handrückens oder der Rückseite der Finger zu kämpfen hat. Da das Kratzen eher von der Unterseite der Fingerspitzen ausgeht, ist es in diesem Fall sinnvoll, wenn die innere Lage die gesamte Hand umfaßt.

Auch bei Kleinkindern kann es wünschenswert sein, wenn die innere Lage das entsprechende Körperteil, beispielsweise das Knie oder den Ellbogen, umfaßt, da dann ein Verrutschen des Schutzbekleidungsartikels um das entsprechend Körperteil herum, so, daß die innere Lage dem betroffenen Bereich, d.h. der Kniebeuge bzw. der Ellbogenbeuge, nicht mehr gegenüberliegt, unkritisch ist.

Die innere Lage besteht vorzugsweise aus einem saugfähigen Gewebematerial aus synthetischen Endlosfasern, wie im europäischen Patent EP 710 303 angegeben. Eine solche innere Lage ist in hohem Maß saugfähig, dennoch in hohem Maß luft- und wasserdampfdurchlässsig. Zur genauen Beschreibung dieses Gewebematerials wird auf den Inhalt der entsprechenden europäischen Patentschrift 710 303 bzw. auf die entsprechende deutsche Patentanmeldung P 43 35 621.4 verwiesen, deren Inhalt zum Bestandteil des Offenbarungsgehaltes dieser Anmeldung gemacht wird.

Solche Materialien haben auch den Vorteil, daß sie Salben und Cremes aufnehmen können, so daß sie auch als Medikamententräger zur Behandlung einer bereits verletzten oder juckenden Körperstelle verwendet werden können. Die äußere Lage besteht vorzugsweise aus einem Gewebe, das eine Bakterienbarriere bildet und flüssigkeitsdicht, jedoch atmungsaktiv ist. Solche Gewebe sind an sich bekannt, beispielsweise in Form des Produktes "Rotecno" (eingetragene Marke) der Firma Rotecno AG, Steinstrasse 35, CH-8045 Zürich. Dieses Gewebe ist ein dichtgewobenes, hydrophobisches Gewebe, das aus endlosen Polyesterfilamenten gewoben ist, beispielsweise unter Anwendung von Microfasern unter 1 Dtex mit mehr als 12000 einzelnen Filamenten pro cm² mit 60 Kettfäden und 60 Schußfäden pro cm, die jeweils aus 144 einzelnen Filamenten bestehen und im Abstand von beispielsweise 5 mm leitfähige Fasern zur Verhinderung von elektrostatischer Aufladung enthalten. Nähere Angaben zu solchen Geweben sind beispielsweise der US-PS 5,335,372 zu entnehmen, deren Inhalt ebenfalls zum Offenbarungsgehalt dieser Patentanmeldung gemacht wird.

Es ist besonders vorteilhaft, wenn die äußere und die innere Lage aus dem gleichen Material, vorzugsweise Polyester, besteht, da einerseits die Entsorgung erleichtert wird (da eine Trennung in einzelne Stoffe entfällt), und weil ein unterschiedliches Schrumpfverhalten beim Waschen, was der Gebrauchsfähigkeit abträglich wäre, nicht befürchtet werden muß.

Besonders bevorzugte Ausführungsformen des erfindungsgemäßen Schutzbekleidungsartikels lassen sich den Unteransprüchen entnehmen.

Die Erfindung wird nachfolgend anhand von Ausführungsbeispielen unter Bezugnahme auf die Zeichnungen näher erläutert, in diesen zeigt:
- Fig. 1: eine Draufsicht auf die eine Seite eines Fäustlings,
- Fig. 1A: eine weitere Ausführungsform des in Fig. 1 gezeigten Fäustlings,
- Fig. 2A-C: Querschnitte durch den Fäustling der Fig. 1, und zwar an den Schnittebenen IIA-IIA, IIB-IIB, bzw. IIC-IIC der Fig. 1,
- Fig. 3: eine Draufsicht auf die andere Seite des Fäustlings der Fig. 1,
- Fig. 4: eine Draufsicht auf die eine Seite einer weiteren Ausführungsform eines erfindungsgemäßen Fäustlings mit getrennten Aufnahmen für den Daumen und den Zeigefinger,
- Fig. 5: eine Draufsicht auf die andere Seite des Fäustlings der Fig. 4,
- Fig. 6: eine Draufsicht auf einen erfindungsgemäßen Bekleidungsartikel zum Schutz der Ellbogenbeuge und zwar auf der Seite, die der Ellbogenbeuge gegenüberliegt,
- Fig. 7: eine Draufsicht auf die Rückseite des erfindungsgemäßen Schutzbekleidungsartikels der Fig. 6,
- Fig. 8: einen schematischen Querschnitt auf der Schnittebene VIII-VIII der Fig. 7,
- Fig. 9A, B: schematisch ein erfindungsgemäßes Bekleidungsoberteil mit einem eingearbeiteten Schutz für den Ellenbogenbereich, sowie eine Seitenansicht des zugehörigen Ärmels veranschaulicht,
- Fig. 10A,B: schematisch eine weitere Ausführungsform eines Bekleidungsoberteiles mit eingearbeitetem Schutz für den Ellenbogenbereich, sowie eine Seitenansicht des zugehörigen Ärmels veranschaulicht,
- Fig. 11: schematisch eine erfindungsgemäße Hose mit eingearbeitetem Schutz für den Kniegelenkbereich in Vorder- und Seitenansicht veranschaulicht,
- Fig. 12: schematisch eine weitere Ausführungsform einer Hose mit eingearbeitetem Schutz für den Kniegelenkbereich in Vorder- und Seitenansicht veranschaulicht,
- Fig. 13, 14: schematisch eine weitere Ausführungsform einer Hose mit eingearbeitetem Schutz für den Kniegelenkbereich in drei Viertel Länge veranschaulichen, und
- Fig. 15: schematisch einen erfindungsgemäßen Strampelanzug veranschaulicht.

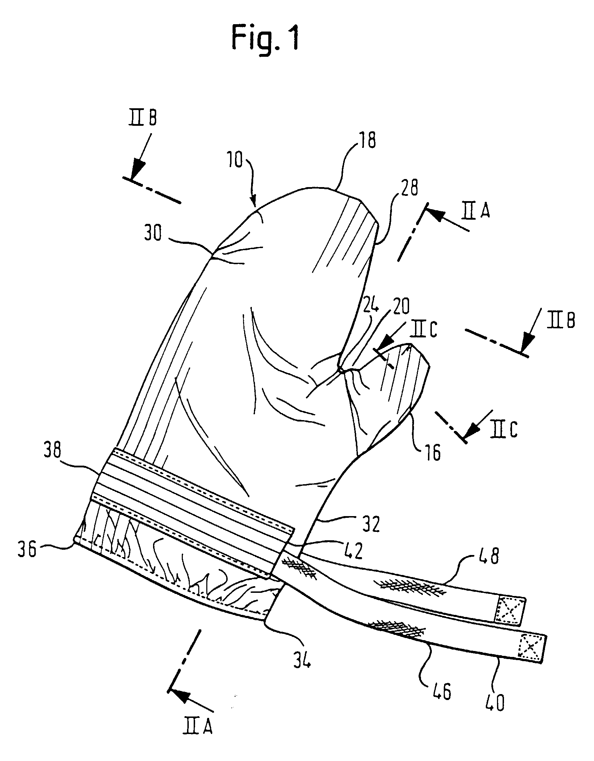

Fig. 1 zeigt einen Schutzbekleidungsartikel 10 in Form eines Fäustlings zur zumindest teilweisen Verhinderung von Kratzverletzungen.

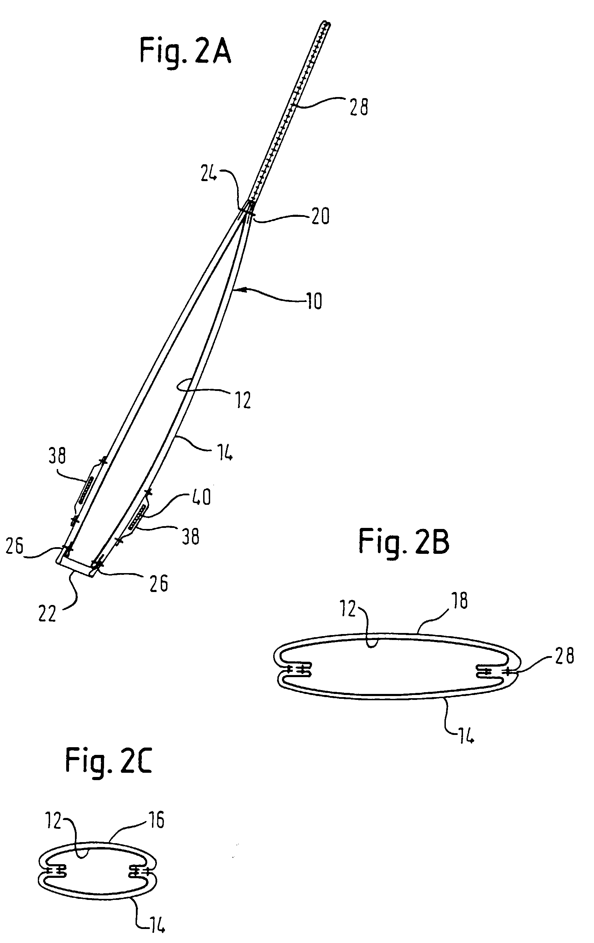

Wie aus der Schnittdarstellung der Fig. 2 hervorgeht, weist der Fäustling der Fig. 1 eine innere Lage 12 und eine äußere Lage 14 auf, die der Einfachheit halber durch einfache Linien in Fig. 2 dargestellt sind. Die innere Lage 12 erstreckt sich durch den gesamten Fäustling, d.h. auch durch den Bereich 16 zur Aufnahme des Daumens und in den Bereich 18 zur Aufnahme der vier Finger hinein. Die innere Lage ist jedoch an die äußere Lage nur im Zwickel 20 zwischen dem Aufnahmebereich 16 und dem Aufnahmebereich 18 und bei der Einführöffnung 22 für die Hand vernäht. Dies bedeutet, daß die zwei Lagen 12 und 14 nur in diesen beiden Bereichen 20 und 22 aneinander befestigt sind, so daß sie in anderen Bereichen gegeneinander verschiebbar sind. Die Stiche im Bereich des Zwickels 20, von denen es nur wenige gibt, sind mit 24 angedeutet. Die entsprechenden Nähte um die Handeinführöffnung 22 herum sind mit 26 angegeben. Die Stiche 24 könnten, falls erwünscht, weggelassen werden, sind jedoch gerade bei Kleinkindern nützlich, da sonst der Daumenbereich der Innenlage dazu neigt, aus dem Aufnahmebereich 16 der Außenlage herausgezogen zu werden und die erneute Einführung sich schwieriger gestaltet als der Fall ist, wenn die Stiche 24 vorgesehen sind.

Fig. 2 zeigt auch eine weitere Naht 28, die nur dazu dient, die zwei übereinander gelegten Hälften der äußeren Lage des Fäustlings zusammenzuhalten. Diese Naht 28 läuft bei der Ausführungsform gemäß Fig. 1 vom Anfang 30 um die Fig. 1 dargestellte äußere Kante des Aufnahmebereiches 18 für die Finger und um die äußere Kante des Aufnahmebereiches 16 für den Daumen und dann entlang der linken Seite 32 des Fäustlings herum bis zum in Fig. 1 untersten Punkt 34 benachbart zur Einführöffnung für die Hand. Der Bereich zwischen dem Punkt 36 und dem Punkt 30, d.h. die linke Kante des Fäustlings in Fig. 1, ist durch eine einfache Faltung des Materials der Außenlage 14 realisiert.

Die innere Lage weist im wesentlichen die gleiche Gestaltung wie die äußere Lage auf mit der gleichen Nahtführung. Wie die innere Lage 12 und die äußere Lage 14 umgeschlagen und aneinander vernäht sind, geht aus den Zeichnungen der Fig. 2A, 2B und 2C hervor.

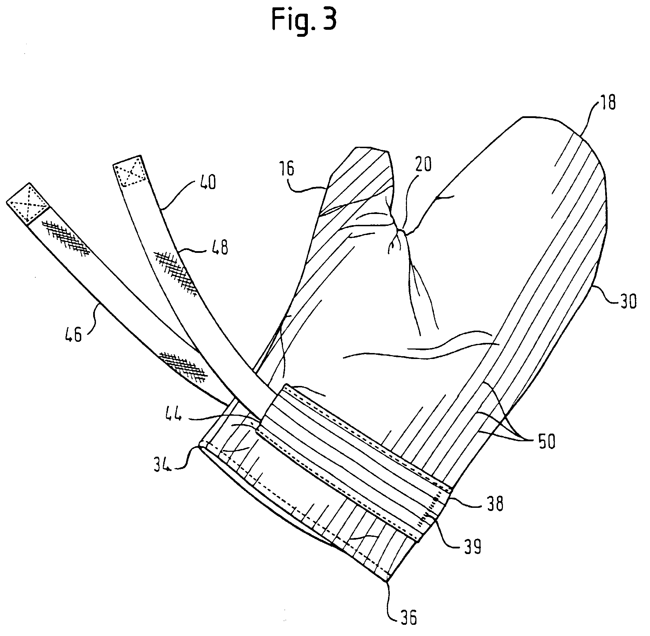

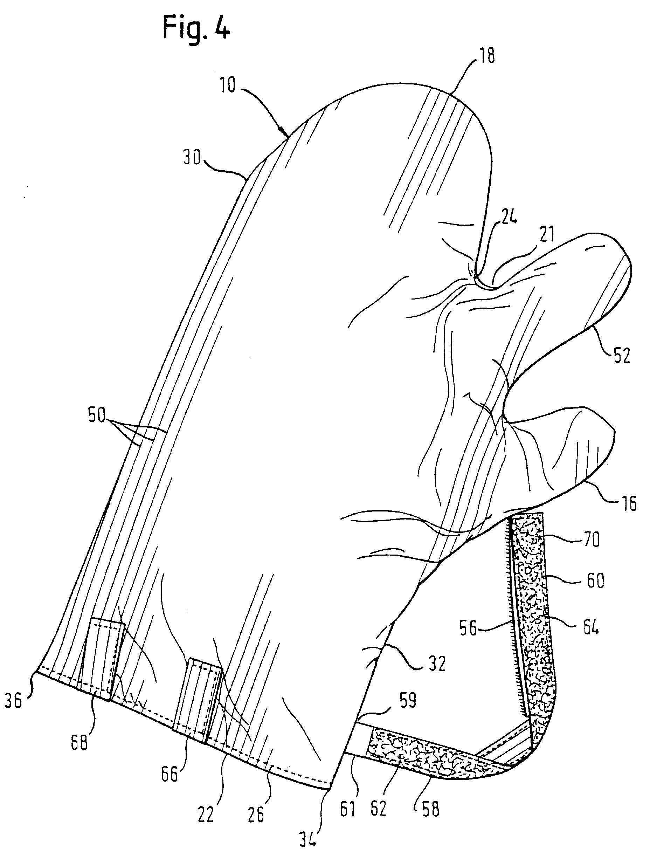

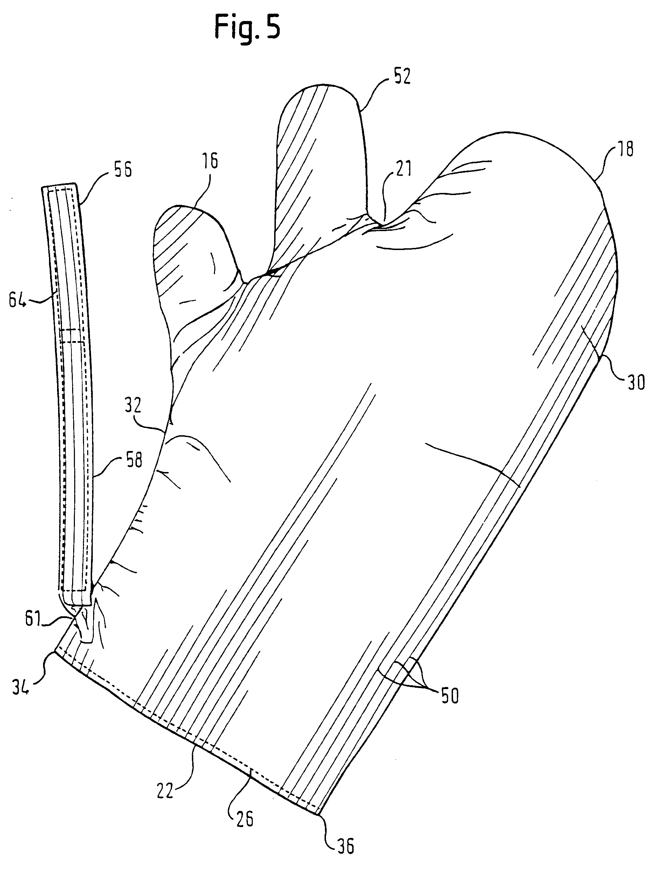

Das Bezugszeichen 38 deutet auf eine zusätzliche Lage des Materials der Außenlage 14 hin, das zur Bildung eines Führungskanals für ein Ziehband 40 dient. Der Führungskanal 38 hat zwei offene Enden bei 42 bzw. 44 (Fig. 3) und wird ebenfalls um die linke Seite des Fäustlings gemäß Fig. 1 gefaltet. Das Ziehband 40, das auch in Form einer Schnur realisiert werden kann, hat zwei freie Enden 46 und 48, die gezogen und beispielsweise in Form einer Schleife miteinander verknotet werden können, um den Fäustling am Handgelenk des Patienten verlustsicher zu befestigen. Anstatt des Bandes 40 könnte auch ein Band mit Klettverschluß zum Einsatz kommen, wie später anhand der Fig. 4 und 5 näher erläutert wird. Um den Verlust des Ziehbandes 40 zu verhindern, kann dieses, wie in Fig. 3 bei 39 gezeigt, im Bereich der linken Kante in Fig. 1 mit dem Handschuh 10 vernäht werden.

Zudem ist es, wie in Fig. 1A gezeigt ist, möglich, daß der Fäustling an der Einführöffnung 22 mit einem Strickbündchen 84 versehen ist. Dieses Strickbündchen 84 besteht vorzugsweise vollständig aus Polyester.

Die innere Lage 12 ist vorzugsweise entsprechend dem EP 710 303 ausgebildet. Das heißt, es besteht aus einem saugfähigen Gewebematerial aus synthetischen Endlosfasern, vorzugsweise aus Polyester. Ein solches Gewebematerial zeichnet sich dadurch aus, daß die Faser des Gewebematerials bauschfähig ist und daß das gewobene Material eine offene Webstruktur aufweist, bei der sich längere, nicht abgebundene Fadenabschnitte mit fest abgebundenen Fadenabschnitten abwechseln.

Die innere Lage könnte aber auch aus einer netzartigen Struktur bestehen. Wesentlich ist, daß die innere Lage aus einem Material besteht, das mit der Haut nicht verklebt, das luftdurchlässig ist und keine Reaktion der Haut verursacht, beispielsweise als hypoallergen gilt. Die Verwendung von synthetischen Endlosfasern bzw. -filamenten ist auch im Hinblick auf eine niedrige Reibung zwischen der Außenlage 14 und der inneren Lage 12 von Bedeutung, da hierdurch die gewünschte erfindungsgemäße Verschiebbarkeit der zwei Schichten besonders gewährleistet ist.

Die äußere Lage 14 besteht vorzugsweise aus einem Gewebe, das eine Bakterienbarriere bildet und flüssigkeitsdicht, jedoch atmungsaktiv ist. Ein solches Gewebe ist beispielsweise das Produkt "Rotecno" (eingetragene Marke) der Firma Rotecno AG, Steinstrasse 35, CH-8045 Zürich. Zum Zwecke der vorliegenden Erfindung wird ein verhältnismäßig leichtes Gewebe dieser Art benutzt. Das entsprechende Gewebe weist auch im Abstand von etwa 5 mm leitfähige Fasern 50 auf, die verhindern, daß Fussel und dergleichen aufgrund von elektrostatischer Ladung vom Gewebe angezogen werden. Dieses Merkmal dient der Aufrechterhaltung der Sterilität des Gewebes, was bei Patienten mit aufgekratzter Haut günstig ist, da hierdurch die Gefahr der Selbstinfektion herabgesetzt werden kann.

Durch die gegenseitige Verschiebbarkeit der inneren und äußeren Lage 12, 14 ist es kaum möglich, daß der Patient durch Fingerbewegungen innerhalb des Fäustlings seine eigene Haut so kratzen kann, daß Hautverletzungen entstehen, da die gegenseitige Verschiebbarkeit der beiden Lagen 12 und 14 als Gleitmittel wirkt und verhindert, daß die Fingerspitzen ausreichend Kraft auf die Haut ausüben können, um solche Kratzverletzungen zu erzeugen.

Bei der Ausführungsform gemäß den Fig. 1 bis 3 umfaßt die innere Lage 12 die gesamte Hand. Dies ist besonders nützlich wenn, wie häufig der Fall ist, der Handrücken vom Patienten durch Kratzen bereits verletzt ist. Versucht der Patient, mit der einen Hand die andere Hand zu kratzen, wird dies insbesondere dann unterbunden, wenn der Patient auf beiden Händen einen jeweiligen Fäustling trägt. Es entsteht sogar ein doppelter Schutz, da nicht nur die Gleitbewegung zwischen der inneren und äußeren Schicht 12 und 14 im Bereich der Finger der einen Hand, sondern auch die gegenseitige Verschiebbarkeit der zwei Lagen im Bereich des Handrückens der anderen Hand tragen dazu bei, die Gefahr von Kratzverletzungen zu reduzieren. Dies gilt auch für den Schutz von anderen Körperteilen, die aufgrund des jeweiligen Krankheitsbilds gefährdet und vorzugsweise mit anderen, erfindungsgemäßen Schutzbekleidungsartikeln geschützt sind.

Da die innere Lage 12 saugfähig ist, kann sie vollflächig oder stellenweise mit einer Heilsalbe oder einer Behandlungssalbe versehen werden, beispielsweise im Bereich des Handrückens, um die entsprechenden Stellen nicht nur zu schützen, sondern auch zu behandeln, um bereits bestehende Verletzungen zu heilen.

Es ist nicht zwingend erforderlich, daß bei einem Fäustling die innere Lage 12 die Hand vollständig umfaßt. In Fällen, wo ein Schutz des Handrückens nicht notwendig ist, da dort beim jeweiligen Patienten keine juckenden Stellen vorliegen, wäre es ausreichend, die innere Lage 12 nur im Fingerbereich vorzusehen. Auch könnte man die äußere Lage 14 ebenfalls nur im Fingerbereich vorsehen und dann etwa im Bereich der Knöchel in Bänder überzuführen, die beispielsweise um das Handgelenk gebunden werden können, um den entsprechenden Schutzbekleidungsartikel an der Hand des Patienten zu befestigen.

Zur Behandlung von bereits verletzten Hautpartien kann auch eine Öffnung vorgesehen werden, so daß beispielsweise eine mit einer Heilsalbe oder mit einem Medikament getränkte Watte zwischen der inneren und äußeren Lage eingesetzt werden kann, so daß die Heilsalbe durch die die Öffnung aufweisende innere Lage an die entsprechenden Hautstellen gelangen kann.

Auch ist es möglich, eine Zwischenlage zwischen der inneren Lage 12 und der äußeren Lage 14 vorzusehen, beispielsweise in Form eines Netzes. Eine solche Zwischenlage könnte der Erhöhung der gegenseitigen Verschiebbarkeit der inneren und äußeren Lage 12, 14 dienen.

Es werden jetzt weitere Ausführungsbeispiele von erfindungsgemäßen Schutzbekleidungsartikeln beschrieben. In den entsprechenden Zeichnungen sind für Teile, die bereits beschriebenen Teilen entsprechen, die gleichen Bezugszeichen verwendet. Es ist davon auszugehen, daß die bisherige Beschreibung auch für die entsprechend numerierten Teile der nachfolgenden Ausführungsform gilt und daß nur besondere Abweichungen näher beschrieben werden.

Bei dem Fäustling der Fig. 4 ist nicht nur ein Aufnahmebereich 16 für den Daumen, sondern auch ein getrennter Aufnahmebereich 52 für den Zeigefinger vorgesehen. Der Aufnahmebereich 18 dient bei dieser Ausführungsform lediglich der Aufnahme der drei verbleibenden Finger, die bei Kratzbewegungen meistens die Hauptrolle spielen. Wenn die innere Lage 12 im Bereich der Finger an der äußeren Lage angenäht ist, erfolgt dies hier im Zwickelbereich 21 zwischen dem Aufnahmebereich 52 für den Zeigefinger und dem Aufnahmebereich 18. Die entsprechenden Stiche 24 erstrecken sich auch hier nur über eine geringe Länge im Bereich des Zwickels 21. Die innere und äußere Lage 12 und 14 sind sonst bei diesem Ausführungsbeispiel genauso ausgebildet wie bei der Ausführung gemäß Fig. 1, nur mit der Ausnahme, daß hier ein Band 56 mit Klettverschluß verwendet wird, um den Fäustling um das Handgelenk zu binden. Das Band 56 hat zwei Bereiche 58 und 60, wobei die zwei Bereiche 58 und 60 zwei miteinander zusammen wirkende Teile eines Klettverschlusses aufweisen. Beispielsweise kann es sich bei dem Bereich 60 um die üblichen hakenförmigen Teile handeln, während im Bereich 58 das filzartige Material eines üblichen Klettverschlusses vorliegt. Diese zwei Teile des Klettverschlusses sind an einem Streifen des Materials der Außenlage 14 vernäht, der an einem Ende bei 59 am Fäustling angenäht ist. Die entsprechenden Nähte sind mit 62 und 64 gekennzeichnet. Anstelle des Führungskanals 38 bei der Ausführungsform gemäß den Fig. 1 bis 3 werden hier nur zwei Schlaufen 66 und 68 vorgesehen. Zur Befestigung des Fäustlings an das Handgelenk wird das freie Ende 70 des Bandes 56 durch beide Schlaufen hindurch geführt, das Band festgezogen und auf sich selbst wieder angedrückt, so daß die Haken im Bereich 60 an einer entsprechenden Stelle entlang des Bereiches 58 haften. Der Bereich 58 könnte in einer Variante anstatt am Band 56 auch an der Außenlage 14 angenäht werden. Auch hier kann der Fäustling sowohl für die linke als auch für die rechte Hand verwendet werden. Des weiteren können die Ausführungsvarianten, die im Zusammenhang mit der Ausführung gemäß Fig. 1-3 beschrieben wurden, auch hier verwendet werden. Auch könnte das Band 56 hier durch die Anordnung der Ziehbänder 46, 48 mit Führungskanal 38 der Ausführungsform gemäß den Fig. 1 bis 3 ersetzt werden.

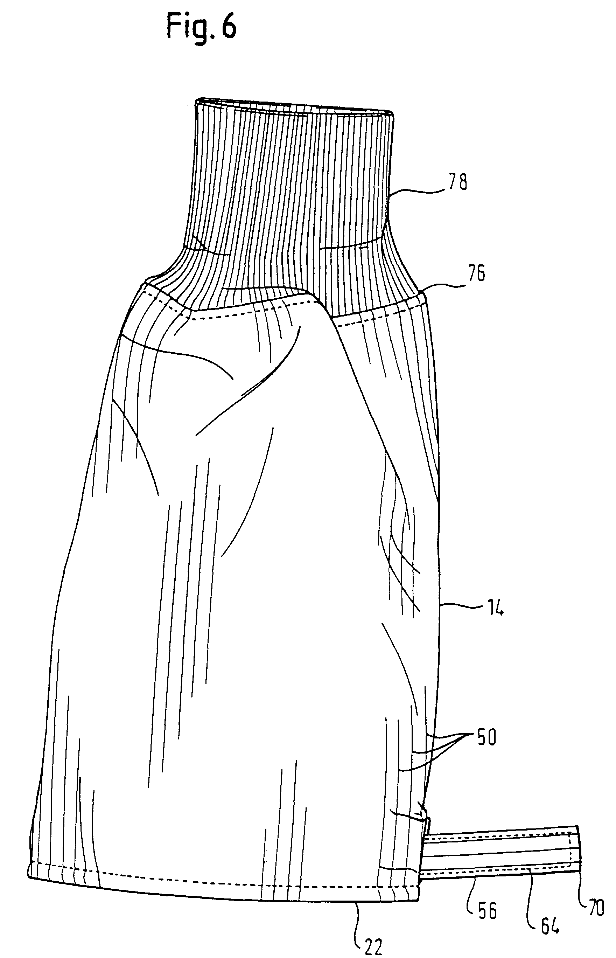

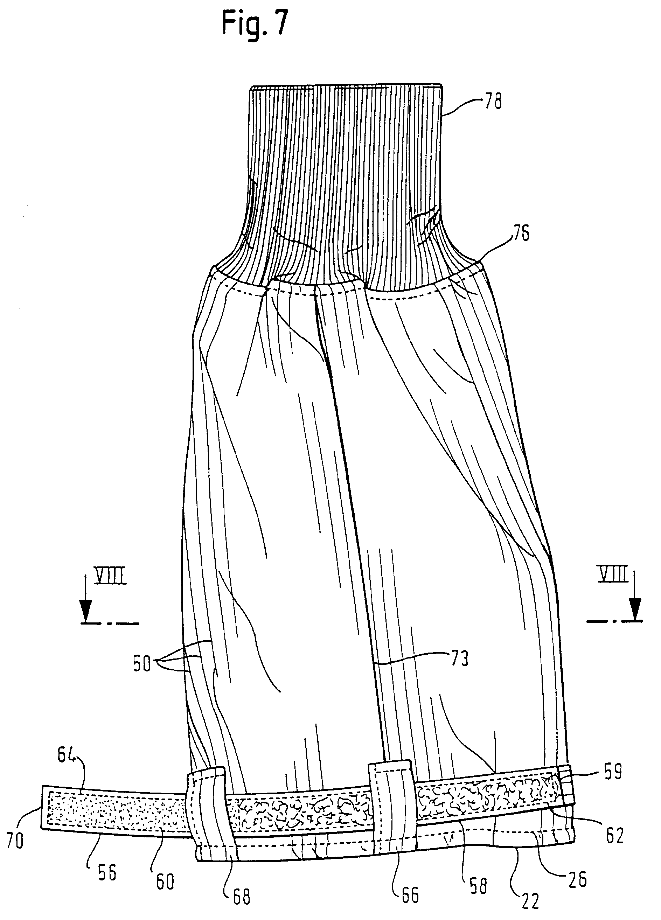

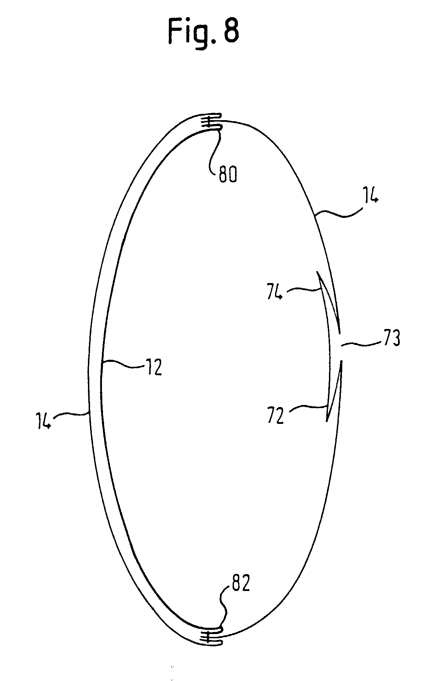

Die Fig. 6 bis 8 zeigen nun einen Bekleidungsartikel zum Schutz der Ellbogenbeuge. Wie insbesondere aus der Querschnittszeichnung der Fig. 8 hervorgeht, weist auch dieser Artikel eine innere Lage 12 und eine äußere Lage 14 auf, wobei die innere Lage 12 im Gebrauch den Ellbogen nicht umfaßt, sondern lediglich auf der inneren Seite des Armes der Ellbogenbeuge gegenüber liegt. Auf der entgegengesetzten Seite des Arms ist lediglich die äußere Lage 14 vorhanden. Diese weist auch eine Doppelfaltung 72, 74 auf, damit der Bekleidungsartikel nicht stramm am Arm des Patienten anliegt. Die einzelnen Falten 72, 74 führen zu einer leicht dehnbaren Fuge 73, die die gegenseitige Verschiebbarkeit der äußeren und inneren Lagen und den Tragekomfort erhöht. Die innere Lage 12 erstreckt sich von der Handeinführöffnung 22 bis zum Übergang 76 zwischen dem Bund 78 und der rohrförmigen Außenlage 14. Die innere Lage 12 ist daher im Bereich der Einführöffnung 22 an seinen beiden Längsrändern 80 und 82 und im Bereich des Überganges zwischen der Außenlage 14 und des Bundes mit der Außenlage 14 bzw. mit der Außenlage und mit dem Bund 78 vernäht.

Es handelt sich bei dem Bund 78 um ein leicht elastisches und gestricktes Teil, das doppelwandig ausgeführt ist. Der Bund besteht ebenfalls aus Polyester und hält das eine Ende des Schutzbekleidungsartikels an der vorgesehenen Stelle im Bereich des Handgelenks. Am entgegengesetzten Ende des rohrförmigen Schutzbekleidungsartikels befindet sich ein Ziehband 56 entsprechend dem Ziehband 56 der Ausführungsform gemäß den Fig. 4 und 5. Auch hier führt das Band 56, das mit zwei Klettverschlußbereichen 58 und 60 versehen ist, und an seinem einen Ende bei 59 mit dem ärmelförmigen Schutzbekleidungsartikel vernäht ist, durch zwei Schlaufen 66 und 68 und kann durch Ziehen und Zurückfaltung an sich selbst befestigt werden. Das Band 56 kommt bei diesem Ausführungsbeispiel oberhalb des Ellbogens, d.h. zwischen dem Ellbogen und der Schulter, zu liegen und hält den Schutzbekleidungsartikel in diesem Bereich fest, so daß die innere Lage wunschgemäß der Ellbogenbeuge gegenüberliegt und nicht verrutschen kann.

Ein weiterer Bereich, der häufig bei Patienten mit Neurodermitis gefährdet ist, ist die Kniebeuge. Zum Schutz der Kniebeuge wird erfindungsgemäß ein Schutzbekleidungsartikel vorgesehen, der in seiner Ausbildung dem Schutzbekleidungsartikel für die Ellbogenbeuge in Form identisch ist, jedoch etwas größere Abmessungen aufweist, um die Anwendung im Bereich der Kniebeuge zu gewährleisten. In diesem Falle liegt der entsprechende Bund 76 im Bereich des Knöchels, während das Band 56 oberhalb des Kniegelenkes positioniert wird und der dortigen Befestigung des Schutzbekleidungsartikels dient.

Auch können sowohl bei dem Schutzbekleidungsartikel für die Ellbogenbeuge wie auch für die Kniebeuge Ausführungsvarianten angewandt werden, die im Zusammenhang mit der ersten erfindungsgemäßen Ausführungsform der Fig. 1 bis 3 beschrieben wurden.

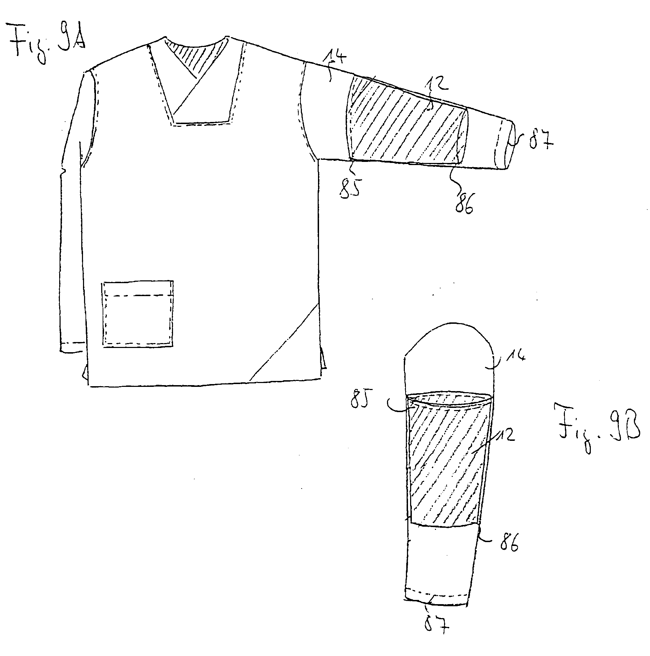

Die Fig. 9 A, B zeigen schematisch einen erfindungsgemäßen Bekleidungsartikel hier in Form eines Bekleidungsoberteils. Hierbei ist der Schutz für die Ellenbogenbeuge gemäß Fig. 6 bis 8 sozusagen in das Bekleidungsoberteil eingearbeitet. Ein Ärmel des Bekleidungsoberteils, der die äußere Lage 14 des Bekleidungsoberteils definiert, ist im Bereich des Ellenbogens mit einer inneren Lage 12 versehen. Die innere Lage 12 ist in diesem Fall rohrförmig ausgebildet und ist nur an der dem freien Ende 87 des Ärmels abgewandten Ende 85 an dem Ärmel befestigt. Die Befestigung erfolgt in diesem Fall durch eine Naht 85, wobei darauf zu achten ist, daß das für die Naht 85 verwendete Material vorzugsweise aus dem gleichen Material wie die innere Lage 12 selbst besteht, um etwaige Hautreizungen der erkrankten Hautpartien durch das Nahtmaterial zu vermeiden. Das dem freien Ende 87 des Ärmels zugewandte Ende 86 der rohrförmigen inneren Lage 12 ist vorzugsweise nicht an dem Ärmel befestigt und liegt daher lose im Ärmel. Dadurch, daß die innere Lage 12 gegenüber dem Ärmel, der hierbei die äußere Lage 14 darstellt, verschiebbar ist, ist sichergestellt, daß bei Kratzbewegungen zwar eine Linderung des Juckreizes erreicht wird, die Gefahr eines Aufkratzens der betreffenden Hautstellen jedoch nicht besteht.

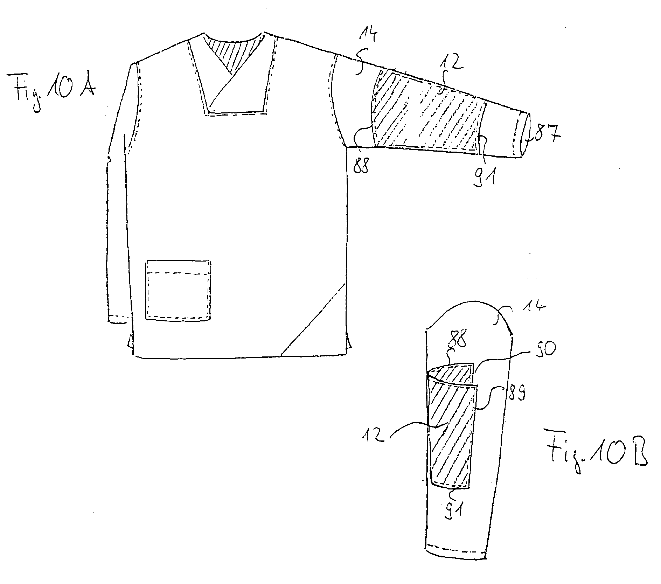

In den Fig. 10 A, B ist schematisch eine weitere Ausführungsform eines erfindungsgemäßen Bekleidungsartikels in Form eines Bekleidungsoberteils ähnlich zu den Fig. 9 A, B gezeigt. Hierbei ist jedoch im Gegensatz zu der Ausführungsform von Fig. 9 die innere Lage 12 nur in einem Teilabschnitt des Ellenbogenbereiches des Ärmels vorgesehen, wobei sie in diesem Fall zum Schutz der Ellenbogenbeuge bestimmt ist. Die Befestigung der inneren Lage 12 an der durch den Ärmel definierten äußeren Lage 14 erfolgt über drei Nähte 88, 89, 90, die an dem dem freien Ende 87 des Ärmels abgewandten Bereich 88, sowie an den mit der Knochenrichtung des Unterarmes ausgerichteten Längsbereichen 89, 90 der inneren Lage 12 vorgesehen sind. Der dem freien Ende 87 des Ärmels zugewandte Bereich 91 der inneren Lage 12 bleibt unbefestigt. Durch die oben beschriebene Befestigung wird die Verschiebbarkeit der inneren Lage 12 gegenüber der äußeren Lage 14 gewährleistet.

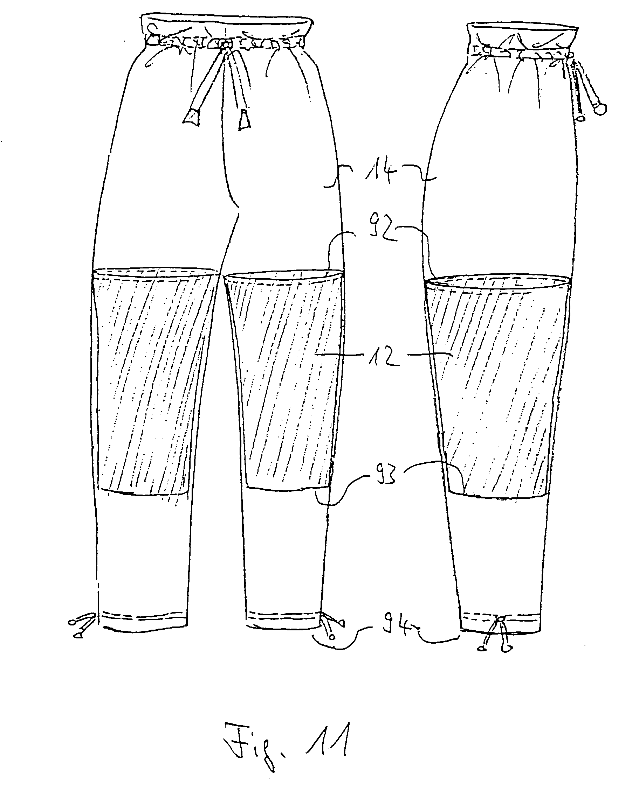

Fig. 11 zeigt schematisch einen erfindungsgemäßen Bekleidungsartikel in Form einer Hose. Hierbei ist ähnlich dem Schutz für die Ellenbogenbeuge gemäß Fig. 6 bis 8 in beide Hosenbeine ein Kratzschutz für die jeweiligen Kniegelenkbereiche sozusagen in das Bekleidungsoberteil eingearbeitet, wie oben bereits beschrieben ist. Die innere Lage 12 ist ähnlich zu Fig. 9 rohrförmig ausgebildet, wobei sie einen größeren Durchmesser als in Fig. 9 aufweist, und ist an dem dem freien Ende 94 des Hosenbeines abgewandten Bereich 92 des Hosenbeines, das die äußere Lage 14 darstellt, befestigt. Somit liegt der dem freien Ende 94 des Hosenbeines zugewandte Bereich 93 der inneren Lage 12 lose in dem Hosenbein, wobei eine gute Verschiebbarkeit der inneren Lage 12 gegenüber der äußeren Lage 14 sichergestellt ist.

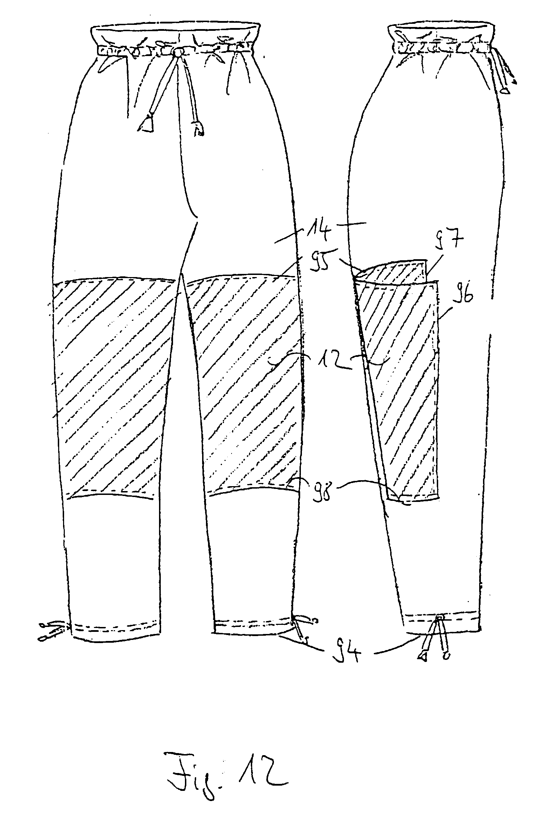

In Fig. 12 ist schematisch eine weitere Ausführungsform eines erfindungsgemäßen Bekleidungsartikels in Form einer Hose ähnlich zu Fig. 11 mit eingearbeitetem Schutz für den Kniegelenkbereich veranschaulicht. Die in eines der beiden Hosenbeine eingearbeitete inneren Lage 12 bedeckt analog zu Fig. 11 nur einen Teilbereich des Kniegelenkbereiches des Hosenbeines und ist hier insbesondere zum Schutz der Kniekehle vorgesehen. Die inneren Lage 12 ist an dem Hosenbein mittels einer dem freien Ende 94 des Hosenbeines abgewandten Quernaht 95 und zweier Längsnähte 96,97 entlang des Hosenbeines befestigt. Der dem freien Ende 94 des Hosenbeines zugewandte Bereich 98 der inneren Lage 12 bleibt unbefestigt.

Es ist auch möglich, daß die erfindungsgemäßen Bekleidungsoberteile und Hosen der Ausführungsformen der Fig. 9 bis 12 auch an anderen als den vorgenannten Stellen oder an zusätzlichen Stellen mit einer inneren Lage 12 versehen sind.

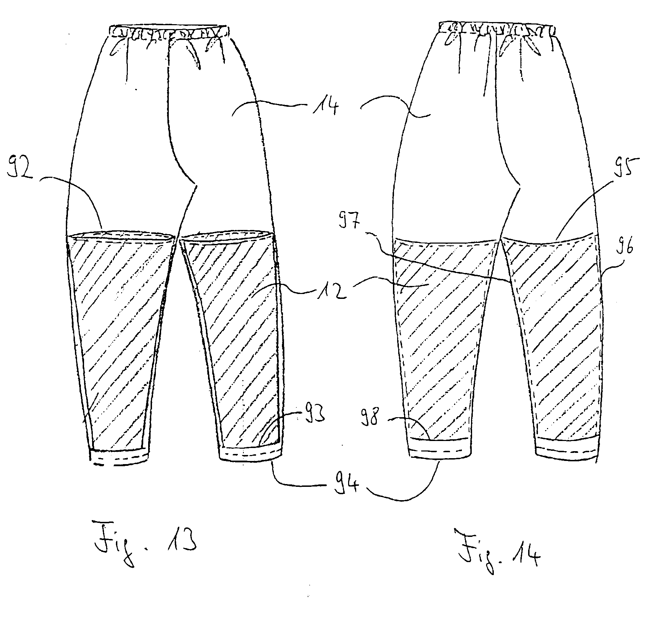

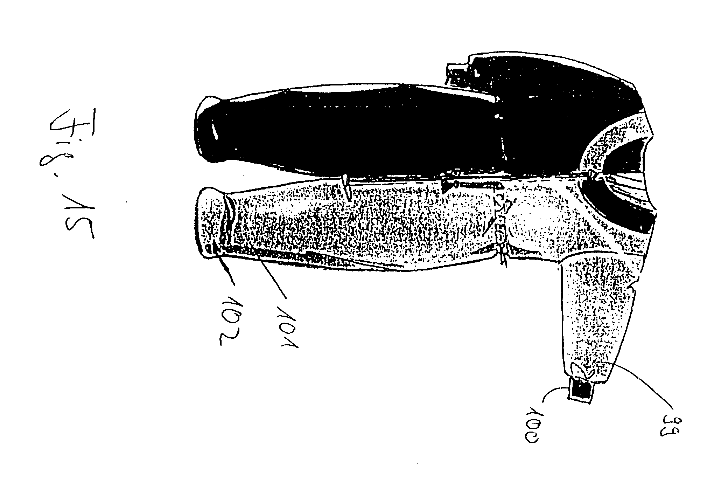

Die in die Bekleidungsoberteile bzw. die Hosen eingearbeitete innere Lage 12 bietet einen vom Design unabhängigen Kratzschutz. Es ist daher beispielsweise auch möglich, die Hosenbeine der erfindungsgemäßen Hose im Gegensatz zu der hier gezeigten Normallänge beispielsweise drei Viertel lang zu gestalten, wie in den Fig. 13, 14 gezeigt ist, ohne den Schutz gegen Aufkratzen an der betreffenden Stelle zu verlieren.

Bei der Befestigung der inneren Lage an den Bekleidungsstücken gemäß der Fig. 9 bis 14 ist darauf zu achten, daß das für die Naht verwendete Material vorzugsweise aus dem gleichen Material wie die innere Lage selbst besteht, um etwaige Hautreizungen der erkrankten Hautpartien durch die Naht zu vermeiden. Es sind auch andere Nahtmaterialien denkbar, sofern sichergestellt ist, das dadurch keinerlei Hautreizungen bewirkt werden können.

Fig. 15 zeigt schematisch einen erfindungsgemäßen Strampelanzug, der eine Kombination der in den Fig. 9 - 14 erläuterten Bekleidungsartikel in Form von Bekleidungsoberteilen und Hosen darstellen kann. Der Strampelanzug kann an beliebigen Stellen, wie beispielsweise an den Ellenbogen- oder Kniekehlenbereichen, mit inneren Lagen 12 versehen sein wie in den Fig. 6 bis 14 gezeigt ist, die beliebig gestaltet sein können, wie beispielsweise rohrförmig oder flach. Bei einer besonders bevorzugten Ausführungsform sind die Ärmelenden 99 mit Strickbündchen 100 oder mit abnehmbaren oder einstückig daran befestigten Fäustlingen gemäß der Fig. 1, 1A, 2 A-C, 3, 4, 5 versehen, um ein Kratzen der erkrankten Hautpartien weiter zu behindern. Zu diesem Zweck kann der erfindungsgemäße Strampelanzug auch einstückig an den Hosenbeinenden 101 befestigte Fußaufnahmen 102 aufweisen.

Die Erfindung ist nicht auf die hier konkret beschriebenen Ausführungsformen begrenzt, sondern es können entsprechende Bekleidungsartikel für alle zu schützenden Körperbereiche konzipiert werden. Wesentlich ist, daß sie im Bereich der zu schützenden Stelle zwei Lagen aufweisen, die gegeneinander verschiebbar sind und vorzugsweise auch die anderen technischen und medizinischen Eigenschaften aufweist, die oben angesprochen sind.

Die gegenseitige Verschiebbarkeit der zwei Lagen, welche insbesondere mit Reibungskoeffizienten unterhalb 0,3, vorzugsweise unterhalb 0,2 und insbesondere unterhalb 0,1 erreicht werden kann, kann beispielsweise sichergestellt werden, wenn die eine Lage aus Strick- oder Wirkware, insbesondere als Schlingenware realisiert wird und die andere Lage gewoben ist, da die gewobene Lage relativ leicht über die Schlingen der Strick-, Wirk- bzw. Schlingenware gleiten kann.

Der Kragenbereich ist auch ein Bereich, der erfindungsgemäß doppellagig mit gegenseitiger Verschiebbarkeit der Lagen ausgeführt werden kann. Insbesondere kann der Kragen hinten relativ weit hochgezogen werden, beispielsweise bis zum breitesten Punkt des Hinterkopfs und nach vorne bis zum Reißverschluß allmählich auslaufen.

Auch ist es erfindungsgemäß günstig, Handschuhe mittels weicher Knöpfe an den Ärmeln eines Bekleidungsoberteils, beispielsweise durch zwei Knöpfe auf der Armunterseite und zwei Knöpfe auf der Armoberseite zu befestigen.

Es soll darauf hingewiesen werden, daß Schutzbekleidungsartikel der angesprochenen Art in Tests mehr als 100 Waschzyklen überstanden haben.

## Patentansprüche

1. Schutzbekleidungsartikel (10) zur zumindest teilweisen Verhinderung von Kratzverletzungen, insbesondere an gefährdeten Körperstellen bei Patienten, vornehmlich bei Kleinkindern und Säuglingen mit Neurodermitis, dadurch gekennzeichnet, daß der Artikel aus einer inneren, der gefährdeten Körperstelle zugewandten Lage (12) und einer äußeren Lage (14) besteht und daß die äußere Lage an der inneren Lage nur an Stellen (24, 26; 76, 80, 82) im Randbereich der inneren Lage fixiert bzw. fixierbar und gegenüber der inneren Lage bei Kratzbewegungen des Patienten verschiebbar ist.

2. Schutzbekleidungsartikel (10) zur zumindest teilweisen Verhinderung von Kratzverletzungen, dadurch gekennzeichnet, daß es sich um einen Handschuh handelt mit einer zumindest im Bereich der Finger vorgesehenen inneren Lage (12) und einer zumindest die Finger und ggf. der Hand umfassenden äußeren Lage (14), und daß die zwei Lagen zumindest im Fingerbereich bei Kratzbewegungen gegeneinander verschiebbar sind.

3. Schutzbekleidungsartikel (10) nach Anspruch 1 und Anspruch 2, dadurch gekennzeichnet, daß eine Befestigungseinrichtung (40, 56) bzw. mehrere Befestigungseinrichtungen (40, 56, 78) vorgesehen ist bzw. sind, um den Artikel (10) an der vorgesehenen Körperstelle zu halten.

4. Schutzbekleidungsartikel (10) nach einem der vorhergehenden Ansprüche, dadurch gekennzeichnet, daß die innere Lage (12) entweder lediglich der entsprechenden Körperstelle (beispielsweise der Ellbogenbeuge) gegenüberliegt, d.h. das entsprechende Körperteil (beispielsweise Ellbogengelenk) nicht umfaßt oder daß die innere Lage (12) der entsprechenden Körperstelle gegenüberliegt und das entsprechende Körperteil umfaßt, wobei die äußere Lage (14) vorzugsweise das die entsprechende Körperstelle aufweisende Körperteil umfaßt.

5. Schutzbekleidungsartikel (10) nach einem der vorhergehenden Ansprüche, dadurch gekennzeichnet, daß zwischen der inneren Lage und der äußeren Lage eine Zwischenlage, beispielsweise in Form eines offenen Netzes vorgesehen ist.

6. Schutzbekleidungsartikel (10) nach einem der vorhergehenden Ansprüche, dadurch gekennzeichnet, daß die innere Lage (12) saugfähig ist und vorzugsweise aus einem saugfähigen Gewebematerial aus synthetischen Endlosfasern besteht.

7. Schutzbekleidungsartikel (10) nach Anspruch 6, dadurch gekennzeichnet, daß es sich bei dem saugfähigen Gewebematerial aus synthetischer Endlosfaser um ein saugfähiges Gewebematerial handelt, das sich dadurch auszeichnet, daß die Faser des Gewebematerials bauschfähig ist und daß das gewobene Material eine offene Webstruktur aufweist, bei der sich längere, nicht abgebundene Fadenabschnitte mit fest abgebundenen Fadenabschnitten abwechseln.

8. Schutzbekleidungsartikel (10) nach einem der vorhergehenden Ansprüche 6 und 7, dadurch gekennzeichnet, daß die innere Lage (12) aus einem Material besteht, das mit der Haut nicht verklebt, das luftdurchlässig ist und keine Reaktion der Haut verursacht, beispielsweise als Hypoallergen gilt, wobei es sich bei dem Material vorzugsweise um Polyester handelt.

9. Schutzbekleidungsartikel (10) nach einem der vorhergehenden Ansprüche, dadurch gekennzeichnet, daß die äußere Lage (14) aus einem Gewebe besteht, das eine Bakterienbarriere bildet und flüssigkeitsdicht, jedoch atmungsfähig ist, vorzugsweise daß es sich bei dem Gewebe um ein dichtgewobenes, hydrophobisches Gewebe handelt, das aus endlosen Polyesterfilamenten gewoben ist, beispielsweise unter Anwendung von Microfasern unter 1 Dtex mit mehr als zwölftausend einzelnen Filamenten pro cm² mit 60 Kettfäden und 60 Schußfäden pro cm, die jeweils aus 144 einzelnen Filamenten bestehen und im Abstand von beispielsweise 5 mm leitfähige Fasern zur Verhinderung von elektrostatischer Aufladung enthalten und beispielsweise daß es sich bei dem Gewebe um das Produkt "Rotecno" (eingetragene Marke) der Firma Rotecno AG, Steinstraße 35, CH-8045 Zürich, handelt.

10. Schutzbekleidungsartikel (10) nach einem der vorhergehenden Ansprüche, dadurch gekennzeichnet, daß zwischen der inneren Lage (12) und der äußeren Lage (14) eine Watte bzw. ein saugfähiges Polster vorgesehen ist, die bzw. das beispielsweise als Heilsalbenträger oder als absorbierendes Medium zur Aufnahme von von der Haut abgesonderten Sekreten dient.

11. Schutzbekleidungsartikel nach einem der vorhergehenden Ansprüche, dadurch gekennzeichnet, daß die innere Lage und die äußere Lage voneinander zerstörungsfrei trennbar und aneinander wieder befestigbar sind, beispielsweise unter Anwendung von Klettverschlüssen.

12. Schutzbekleidungsartikel (10) nach einem der vorhergehenden Ansprüche, dadurch gekennzeichnet, daß er als Fäustling ausgebildet ist, wobei die innere Lage (12) die Hand umfaßt und im Bereich der Handeinführöffnung (22) an der äußeren Lage (14) befestigt ist, wahlweise daß der Fäustling ohne Daumenteil ausgebildet ist, d.h. die vier Finger und der Daumen der Hand in einem umfaßt oder ebenfalls wahlweise daß der Fäustling eine getrennte Aufnahme (16) für den Daumen aufweist, wobei vorzugsweise die innere Lage (12) an der äußeren Lage (14) im Bereich des Übergangs (20) von der Aufnahme (16) für den Daumen zur Aufnahme (18) für die Finger befestigt ist.

13. Schutzbekleidungsartikel (10) nach Anspruch 12, dadurch gekennzeichnet, daß der Fäustling Aufnahmebereiche (16, 52) für den Daumen und für den Zeigefinger aufweist und daß die innere Lage (12) an der äußeren Lage (14) im Bereich des Überganges (21) von der Aufnahme (52) für den Zeigefinger zu der Aufnahme (18) für die verbleibenden Finger der Hand aufweist, wobei gegebenenfalls die äußere Lage (14) die innere Lage (12) und die Hand voll umfaßt und mittels Ziehschnüren bzw. -bänder (46, 48) am Handgelenk befestigbar ist oder daß die Befestigungseinrichtung am Handgelenk vorgesehen ist und aus einem Band (56) besteht, das durch Schlaufen (66, 68) im Handgelenkbereich des Handschuhs bzw. des Fäustlings hindurch geführt wird und festziehbar und mit sich selbst bzw. mit einem Gegenstück an der äußeren Lage (14) befestigbar ist, wobei vorzugsweise das Band (56) an sich selbst oder an der äußeren Lage über einen Klettverschluß (58, 60) befestigbar ist.

14. Schutzbekleidungsartikel (10) nach einem der Ansprüche 1 und 3 bis 11, dadurch gekennzeichnet, daß es sich um einen Artikel zum Schutz der Ellbogenbeuge handelt, wobei er vorzugsweise rohrförmig ausgebildet ist, daß die innere Lage (12) und die äußere Lage (14) an der Handgelenkseite an einem Bund (78) befestigt sind und daß am anderen Ende der Artikel mit einer oberhalb des Ellbogens zur Einsatz kommenden Befestigungseinrichtung (56) ausgestattet ist.

15. Schutzbekleidungsartikel (10) nach Anspruch 14, dadurch gekennzeichnet, daß die innere Lage (12) der Ellbogenbeuge zugewandt angeordnet ist und weder den Unterarm noch den Ellbogen umfaßt, vorzugsweise daß die innere Lage (12) an ihren Längsrändern (80, 82), d.h. die Ränder, die zumindest im wesentlichen parallel zur Knochenrichtung des Unterarms verlaufen, an der äußeren Lage (14) befestigt bzw. befestigbar sind, wobei beispielsweise die Befestigungseinrichtung oberhalb des Ellbogens durch Ziehschnüre bzw. - bänder gebildet ist.

16. Schutzbekleidungsartikel (10) nach einem der Ansprüche 14 oder 15, dadurch gekennzeichnet, daß die Befestigungseinrichtung aus einem Band (56) besteht, das durch Schlaufen (66, 68) oberhalb des Ellbogens hindurch geführt wird und festziehbar und mit sich selbst bzw. mit einem Gegenstück an der äußeren Lage (14) befestigbar ist, wobei das Band (56) gegebenenfalls an sich selbst oder an der äußeren Lage (14) über einen Klettverschluß (58, 60) befestigbar ist.

17. Schutzbekleidungsartikel (10) nach einem der vorhergehenden Ansprüche 1 bzw. 3 bis 11, dadurch gekennzeichnet, daß es sich um einen Artikel zum Schutz der Kniebeuge handelt, wobei er vorzugsweise rohrförmig ausgebildet ist, daß die innere Lage (12) und die äußere Lage (14) an der Fußgelenkseite an einem Bund (78) befestigt sind und daß am anderen Ende der Artikel mit einer oberhalb des Knies zur Einsatz kommenden Befestigungseinrichtung ausgestattet ist.

18. Schutzbekleidungsartikel (10) nach Anspruch 17, dadurch gekennzeichnet, daß die innere Lage (12) der Kniebeuge zugewandt angeordnet ist und weder den Unterschenkel noch das Kniegelenk umfaßt, vorzugsweise daß die innere Lage (12) an ihren Längsrändern (80, 82), d.h. die Ränder, die zumindest im wesentlichen parallel zur Knochenrichtung des Unterschenkels verlaufen, an der äußeren Lage (14) befestigt bzw. befestigbar sind, wobei beispielsweise die Befestigungseinrichtung oberhalb des Knies durch Ziehschnüre bzw. - bänder gebildet ist.

19. Schutzbekleidungsartikel (10) nach einem der Ansprüche 17 oder 18, dadurch gekennzeichnet, daß die Befestigungseinrichtung aus einem Band (56) besteht, das durch Schlaufen (66, 68) oberhalb des Knies hindurch geführt wird und festziehbar und mit sich selbst bzw. mit einem Gegenstück an der äußeren Lage (14) befestigbar ist, wobei das Band (56) an sich selbst oder an der äußeren Lage über einen Klettverschluß (58, 60) befestigbar ist.

20. Schutzbekleidungsartikel (10) nach einem der Ansprüche 1, 3 sowie 6 - 11, dadurch gekennzeichnet, daß er als ein Bekleidungsoberteil mit zwei Ärmeln, insbesondere als ein Haus- bzw. Schlafanzugoberteil, ausgebildet ist.

21. Schutzbekleidungsartikel (10) nach Anspruch 4 und 20, dadurch gekennzeichnet, daß in zumindest einem der Ärmel des Bekleidungsoberteiles die innere Lage (12) nur der zu schützenden Körperstelle, insbesondere der Ellenbogenbeuge, zugeordnet ist und somit den Ellenbogenbereich nur teilweise umgibt, wobei sie mit ihrem dem freien Ende (87) des Ärmels abgewandten, quer zur Knochenrichtung des Unterarmes verlaufenden Rand (88) und ihren beiden Längsränder (89, 90) entlang der Knochenrichtung des Unterarmes im Ellenbogenbereich des Ärmels befestigt bzw. befestigbar ist und der dem freien Ende (87) des Ärmels zugewandte Rand (91) der inneren Lage (12) vorzugsweise unbefestigt bleibt.

22. Schutzbekleidungsartikel (10) nach Anspruch 5 und 20, dadurch gekennzeichnet, daß in zumindest einem der Ärmel des Bekleidungsoberteiles die innere Lage (12) den zu schützenden Bereich, insbesondere den Ellenbogenbereich, im Ärmel rohrförmig umgibt, wobei sie mit ihrem dem freien Ende (87) des Ärmels abgewandten Rand (85) an dem Ärmel befestigt bzw. befestigbar ist, und der dem freien Ende (87) des Ärmels zugewandte Rand (86) der inneren Lage (12) vorzugsweise unbefestigt bleibt.

23. Schutzbekleidungsartikel (10) nach einem der Ansprüche 1, 3 sowie 6 - 11, dadurch gekennzeichnet, daß er als eine Hose mit zwei Hosenbeinen, insbesondere als eine Haus- bzw. Schlafanzughose, ausgebildet ist.

24. Schutzbekleidungsartikel (10) nach Anspruch 4 und 23, dadurch gekennzeichnet, daß in zumindest einem der Hosenbeine der Hose die innere Lage (12) nur der zu schützenden Körperstelle, insbesondere der Kniegelenkbeuge, zugeordnet ist und somit den Kniegelenkbereich nur teilweise umgibt, wobei sie mit ihrem dem freien Ende (94) des Hosenbeines abgewandten, quer zur Knochenrichtung des Unterschenkels verlaufenden Rand (95) und ihren beiden Längsränder (96, 97) entlang der Knochenrichtung des Unterschenkels im Kniegelenkbereich des Hosenbeines befestigt bzw. befestigbar ist und der dem freien Ende (94) des Hosenbeines zugewandte Rand (98) der inneren Lage (12) vorzugsweise unbefestigt bleibt.

25. Schutzbekleidungsartikel (10) nach Anspruch 4 und 23, dadurch gekennzeichnet, daß in zumindest einem der Hosenbeine der Hose die innere Lage (12) den zu schützenden Bereich, insbesondere den Kniegelenkbereich, im Hosenbein rohrförmig umgibt, wobei sie mit ihrem dem freien Ende (94) des Hosenbeines abgewandten Rand (92) an dem Hosenbein befestigt bzw. befestigbar ist, und der dem freien Ende (94) des Hosenbeins zugewandte Rand (93) der inneren Lage (12) vorzugsweise unbefestigt bleibt.

26. Schutzbekleidungsartikel (10), dadurch gekennzeichnet, daß er als ein Overall, insbesondere als ein Strampelanzug, ausgebildet ist, der eine Kombination eines Bekleidungsoberteils nach Anspruch 20 bis 22 und einer Hose nach Anspruch 23 bis 25 umfaßt.

27. Schutzbekleidungsartikel (10) nach Anspruch 26, dadurch gekennzeichnet, daß die Enden (99) der Ärmel mit Strickbündchen (100) oder mit Fäustlingen versehen sind.

28. Schutzbekleidungsartikel (10) nach Anspruch 26, dadurch gekennzeichnet, daß die Enden (101) der Hosenbeine mit mit dem Overall einstückig ausgebildeten Fußaufnahmen (102) versehen sind.
